# EUROPEAN PATENT APPLICATION

(11) **EP 1 374 870 A1**
(43) Date of publication of application: **02.01.2004**
(21) Application number: 03013434.0
(22) Date of filing: 23.06.2003
(51) Int. Cl.: A61K 31/517, A61K 31/415, A61K 31/40, A61K 31/54, A61K 31/18, A61K 31/42, A61K 31/34, A61P 25/06

(54) **Medication for the treatment or prevention of migraine**

(30) Priority: 24.06.2002 EP 02013882
(71) Applicant: Dobmeyer, Rita, 60528 Frankfurt (DE)
(72) Inventor: Dobmeyer, Rita, 60528 Frankfurt (DE)

(57) **Abstract**

The invention relates to a medicamentation for the treatment of prevention of migraine comprising an inhibitor of CGRP (**1**) and at least one cyclooxygenase-2 inhibitor or a pharmaceutically acceptable salt, solvate, or physiologically functional derivative thereof (**2**), and a pharmaceutically acceptable carrier or excipient, and optionally one or more other therapeutic ingredients.

## Description

### FIELD OF THE INVENTION

The invention relates to a medicamentation for the treatment of prevention of migraine comprising an inhibitor of CGRP (**1**) and at least one cyclooxygenase-2 inhibitor or a pharmaceutically acceptable salt, solvate, or physiologically functional derivative thereof (**2**), and a pharmaceutically acceptable carrier or excipient, and optionally one or more other therapeutic ingredients.

### BACKGROUND OF THE INVENTION

CGRP is a 37 amino acid polypeptide that is stored and released from nerve terminals in both the central nervous system and the peripheral nervous system.

(Goodman et al., Life Sci., Vol. 38, pp. 2169-2172 (1986)). CGRP has been detected in nerves innervating the heart, peripheral and cerebral blood vessels, and kidneys by immunohistochemical and radioimmunoassay methods. (Yamamoto et al., Prog. Neurobiol., Vol. 33, pp. 335-386 (1989)). CGRP has been shown to mediate its biological response by binding to specific cell surface receptors that have been identified in a variety of tissues. Evidence from biochemical studies suggest that CGRP receptors belong to the family of G-protein coupled receptors. The widespread distribution of CGRP receptors on muscle, glandular, epithelial and neuronal cells is consistent with its wide range of biological actions, including peripheral and cerebral vasodilation (Brain et al., Nature, Vol. 313, pp. 54-56 (1985)); cardiac acceleration (Sigrist et al., Endocrinology, Vol. 119, pp. 381-389 (1986)); regulation of calcium metabolism (Grunditz et al., Endocrinology, Vol. 119, pp. 2313-2324 (1986)); reduction of intestinal motility (Fargeas et al., Peptides, Vol. 6, pp. 1167-1171(1985)); regulation of glucose metabolism, e.g., reduction of insulin secretion and insulin sensitivity, (Hermansen et al., Peptides, Vol. 27, pp. 149-157 (1990); and Molina et al., Diabetes, Vol. 39, pp. 260-265 (1990)); reduction of appetite and reduction of growth hormone increase (Tannenbaum et al., Endocrinology, Vol. 116, pp. 2685-2687 (1985)).

Since CGRP has a number of effects on the cardiovascular, central nervous, gastrointestinal, respiratory and endocrine systems, it has now been discovered that limited and selective inhibition of CGRP receptor mechanisms represents a novel preventative and therapeutic approach to the treatment of a broad variety of disease states that are mediated by CGRP. In particular, the development of an active CGRP receptor antagonist would be expected to be useful in the treatment of a variety of disease states that are mediated by CGRP including, but not limited to, headaches, especially migraines; NIDDM; cardiovascular disorders; chronic inflammation; endotoxic shock; arthritis; allergic rhinitis; and asthma, all in mammals, preferably humans.

Unfortunately, there is a high rate of recurrence of symptoms, especially if patients suffering from migraine are treated with inhibitors of CGRP.

Surprisingly, it has been found that this recurrence can be suppressed, when such patients are treated with a cyclooxygenase inhibitor in addition to the CGRP inhibitor.

### SUMMARY OF THE INVENTION

In one aspect the present invention relates to a medicamentation comprising an antagonist, of the Calcitonin Gene-Related Peptide (CGRP) (**1**)and at least one cyclooxygenase-2inhibitor or at least one combined cyclooxygenase-1 / cyclooxygenase-2 (cox1/cox2) inhibitor or a pharmaceutically acceptable salt, solvate, or physiologically functional derivative thereof (**2**), and a pharmaceutically acceptable carrier or excipient, and optionally one or more other therapeutic ingredients.

In another aspect, the present invention the present invention relates to a method for the prevention or treatment migraine which method comprises administration of effective amounts of an inhibitor of CGRP (**1**) and a cyclooxygenase-2 or combined cox1/coxII inhibitor (**2**) to a patient in need thereof in a combined form, or separately or separately and sequentially wherein the sequential administration is close in time or remote in time.

In yet another aspect, the present invention relates to the use of an inhibitor of CGRP (**1**) and a cyclooxygenase-2 inhibitor (**2**) in a combined form, or separately or separately and sequentially, wherein the sequential administration is close in time or remote in time, for the manufacture of a medicamentation for the prevention or treatment of migraine, in particular for the prevention of an early recurrence of migraine.

Another aspect of the present invention is a pharmaceutical kit comprising at least two separate unit dosage forms (A) and (B):
(A) one of which comprises a composition containing an inhibitor of CGRP and optionally a pharmaceutically acceptable carrier;
(B) one of which comprises a composition containing one or more cyclooxygenase-2 inhibitors, and optionally a pharmaceutically acceptable carrier.

### DETAILED DESCRIPTION OF THE INVENTION

Inhibitors of CGRP are known for example from International Patent applications WO98/09630 WO 98/11128, WO 98/56779, WO 00/18764, WO 00/55154, WO 01/149676, WO 01/32648 and WO 01/32649. Particularly preferred is a compound of formula 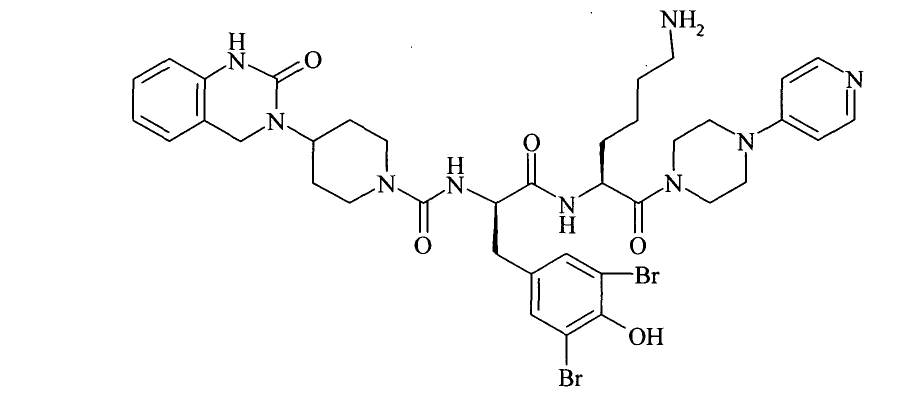 which can be prepared as described in the International patent application WO 98/11128.

Suitable COX-2 or COX1 / COX2 inhibitors (**2**) are for example celecoxib, Dupont Dup 697, etodolac, etoricoxib, flosulide, meloxicam, nimesulide, parecoxib, rofecoxib, Taisho NS-398 and valdecoxib, most preferably meloxicam of formula 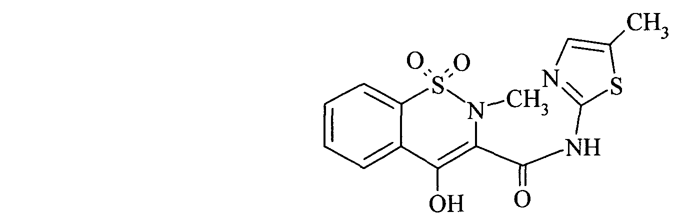 which is known for example from German patent application DE 27 56 113.

The ratio of (**1**) to (**2**) in the medicamentations according to the invention is as a rule in the range of 50:1 to 1:300, preferably in the range 8:1 to 1:80, in particular in the range of about 1:2 to 1:3.

For example, without restricting the scope of the invention thereto, preferred combinations of (**1**) and (**2**) according to the invention may contain (**1**) and (**2**) in the following weight ratios: 1:50; 1:49; 1:48; 1:47; 1:46; 1:45; 1:44; ₁:43; 1:42; 1:41; 1:40; 1:39; 1:38; 1:37; 1:36; 1:35; 1:34; 1:33; 1:32; 1:31; 1:30; 1:29; 1:28; 1:27; 1:26; 1:25; 1:24; 1:23; 1:22; 1:21; 1:20; 1:19; 1:18; 1:17; 1:16; 1:15; 1:14; 1:13; 1:12; 1:11; 1:10; 1:9; 1:8; 1:7; 1:6; 1:5; 1:4; 1:3; 1:2; 1:1; 2:1; 3:1; 4:1; 5:1.

The medicamentation according to the invention comprising the combinations of (**1**) and (**2**) are normally administered so that (**1**) and (**2**) are present together in doses of 0.1 to 10,000 µg, preferably from 2 to 2000 µg, more preferably from 2 to 1000 µg, better still from 5 to 500 µg, preferably, according to the invention, from 10 to 300 µg, better still 20 to 200 µg per single dose.

A suitable daily dose of (**1**) is in the range 10 to 400 µg, in particular 20 to 200 µg. A suitable daily dose of (**2**) is in the range 50 µg to 2000 µg, in particular 75 to 1000 µg. Both the doses of (1) and of (**2**) are depending on the type of migraine to be treated, the severity of the disease, the age, weight and condition of the patient. Further the dose of (1) will be dependent on the salt form used (or base), whether racemate or enriched in an enantiomeric form.

The medicamentation according to present invention is suitable for oral, inhalativ, intravascular, intraperitoneal, subcutaneous, intramuscular or topical administration.

The active substance **1** is preferably administered by injection or inhalation. For this purpose, ingredient **1** has to be made available in forms suitable for injection or inhalation.

Inhalable preparations include inhalable powders, propellant-containing metering aerosols or propellant-free inhalable solutions. Inhalable powders according to the invention containing the active substance **1** may consist of the active substance on its own or of a mixture of the active substances with physiologically acceptable excipients. Within the scope of the present invention, the term propellant-free inhalable solutions also includes concentrates or sterile inhalable solutions ready for use. The preparations according to the invention may contain the combination of active substances **1** and **2** either together in one formulation or in two separate formulations. These formulations which may be used within the scope of the present invention are described in more detail in the next part of the specification.

### A) Inhalable powder containing the active substance 1 according to the invention:

The inhalable powders according to the invention may contain **1** either on its own or in admixture with suitable physiologically acceptable excipients.

If the active substance **1** is present in admixture with physiologically acceptable excipients, the following physiologically acceptable excipients may be used to prepare these inhalable powders according to the invention: monosaccharides (e.g. glucose or arabinose), disaccharides (e.g. lactose, saccharose, maltose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose is the particularly preferred excipient, while lactose monohydrate is most particularly preferred.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipient mentioned above. These finer excipients are also selected from the group of possible excipients listed hereinbefore. Finally, in order to prepare the inhalable powders according to the invention, micronised active substance **1** is added to the excipient mixture. Processes for producing the inhalable powders according to the invention by grinding and micronising and by finally mixing the ingredients together are known from the prior art.

The inhalable powders according to the invention may be administered using inhalers known from the prior art. Inhalable powders according to the invention which contain a physiologically acceptable excipient in addition to **1** may be administered, for example, by means of inhalers which deliver a single dose from a supply using a measuring chamber as described in US 4,570,630, or by other means as described in DE 36 25 685 A. Preferably, the inhalable powders according to the invention which contain physiologically acceptable excipient in addition to **1** are packed into capsules (to produce so-called inhalettes) which are used in inhalers as described, for example, in WO 94/28958.

If the inhalable powders according to the invention are packed into capsules (inhalers) for the preferred use described above, the quantities packed into each capsule should be 1 to 30mg, preferably 3 to 20mg, more particularly 5 to 10mg of inhalable powder per capsule.

### B) Propellant gas-driven inhalation aerosols containing the active substance 1:

Inhalation aerosols containing propellant gas according to the invention may contain substance **1** dissolved in the propellant gas or in dispersed form. **1** may be present in separate formulations or in a single preparation, in which **1** is either dissolved or dispersed. The propellant gases which may be used to prepare the inhalation aerosols according to the invention are known from the prior art. Suitable propellant gases are selected from among hydrocarbons such as n-propane, n-butane or isobutane and halohydrocarbons such as fluorinated derivatives of methane, ethane, propane, butane, cyclopropane or cyclobutane. The propellant gases mentioned above may be used on their own or in mixtures thereof. Particularly preferred propellant gases are halogenated alkane derivatives selected from TG134a and TG227. Of the halogenated hydrocarbons mentioned above, TG134a (1,1,1,2-tetrafluoroethane) and TG227 (1,1,1,2,3,3,3-heptafluoropropane) and mixtures thereof are preferred according to the invention.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **1**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1 wt.-% of active substance **1**.

The propellant-driven inhalation aerosols according to the invention mentioned above may be administered using inhalers known in the art (MDIs = metered dose inhalers). Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are characterised in that they contain the propellant gas-containing aerosols described above according to the invention. The present invention also relates to cartridges which are fitted with a suitable valve and can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### C) Propellant-free inhalable solutions or suspensions containing the active substance 1 according to the invention:

It is particularly preferred to use the active substance combination according to the invention in the form of propellant-free inhalable solutions and suspensions. The solvent used may be an aqueous or alcoholic, preferably an ethanolic solution. The solvent may be water on its own or a mixture of water and ethanol. The relative proportion of ethanol compared with water is not limited but the maximum is up to 70 percent by volume, more particularly up to 60 percent by volume and most preferably up to 30 percent by volume. The remainder of the volume is made up of water. The solutions or suspensions containing **1** are adjusted to a pH of 2 to 7, preferably 2 to 5, using suitable acids. The pH may be adjusted using acids selected from inorganic or organic acids. Examples of suitable inorganic acids include hydrochloric acid, hydrobromic acid, nitric acid, sulphuric acid and/or phosphoric acid. Examples of particularly suitable organic acids include ascorbic acid, citric acid, malic acid, tartartic acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavorings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium editate, as stabilizer or complexing agent is unnecessary in the present formulation. Other embodiments may contain this compound or these compounds. In a preferred embodiment the content based on sodium editate is less than 100mg/100ml, preferably less than 50mg/ml, more preferably less than 20mg/ml. Generally, inhalable solutions in which the content of sodium editate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents.

The preferred excipients include antioxidants such as ascorbic acid, for example, provided that it has not already been used to adjust the pH, vitamin A, vitamin E, tocopherols and similar vitamins and pro-vitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50uL, more preferably between 10 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.
Preferred devices for delivering the device are a metered dose inhaler containing an appropriate propellant such as 1,1,1,2-Tetrafluoroethane, a powder inhaler such as HandiHaler®, or a nebulizer or soft mist inhaler such as described in WO 91/14468 or figures 6a and 6b of WO 97/12687.

The active substance **2** according to the invention is preferably administered orally or by suppository formulations. For this purpose, ingredient **2** has to be made available in forms suitable for oral administration or suppository formulation.

Suitable preparations include for example tablets, capsules, suppositories, solutions, elixirs, emulsions or dispersible powders.

Suitable tablets may be obtained, for example, by mixing the active substance with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

## Claims

1. A medicamentation comprising an antagonist, of the Calcitonin Gene-Related Peptide (CGRP) (**1**)and at least one cyclooxygenase-2inhibitor or at least one combined cyclooxygenase-1 / cyclooxygenase-2 (cox1/cox2) inhibitor or a pharmaceutically acceptable salt, solvate, or physiologically functional derivative thereof (**2**), and a pharmaceutically acceptable carrier or excipient, and optionally one or more other therapeutic ingredients.

2. A medicamentation according to claim 1 consisting essentially of BIBN 4096 (**1**) and one cyclooxygenase-2 inhibitor or combined cox1/coxII inhibitor selected from the group consisting of celecoxib, Dupont Dup 697, etodolac, etoricoxib, flosulide, meloxicam, nimesulide, parecoxib, rofecoxib, Taisho NS-398 and valdecoxib, or a pharmaceutically acceptable salt, solvate, or physiologically functional derivative thereof (**2**), and a pharmaceutically acceptable carrier or excipient.

3. A medicamentation according to claim 1 or 2 comprising the BIBN 4096 (**1**) and meloxicam of formula 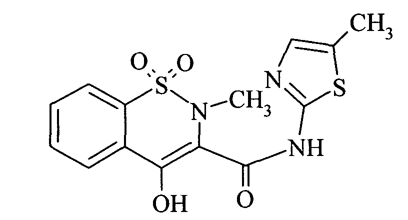 or a pharmaceutically acceptable salt thereof (**2**), and a pharmaceutically acceptable carrier or excipient.

4. A medicamentation according to any of claims 1 to 3 which is suitable for oral, inhalativ, intravascular, intraperitoneal, subcutaneous, intramuscular or topical administration.

5. A medicamentation according to any of claims 1 to 4 wherein the weight ratio of (**1**) to (**2**) ranges from 50:1 to 1 : 300, preferably from 8:1 to 1:80.

6. A medicamentation according to any of claims 1 to 5 wherein a single application dose contains 1 to 10,000 milligrams, preferably 10 to 2,000 mg of the combined active ingredients (**1**) and (**2**).

7. A method for the prevention or treatment migraine which method comprises administration of effective amounts of an inhibitor of CGRP (**1**) and a cyclooxygenase-2 or combined cox1/coxII inhibitor (**2**) to a patient in need thereof in a combined form, or separately or separately and sequentially wherein the sequential administration is close in time or remote in time.

8. Use of an inhibitor of CGRP (**1**) and a cyclooxygenase-2 inhibitor (**2**) in a combined form, or separately or separately and sequentially, wherein the sequential administration is close in time or remote in time, for the manufacture of a medicamentation for the prevention or treatment of migraine.

9. Use of an inhibitor of CGRP (**1**) and a cyclooxygenase-2 inhibitor (**2**) in a combined form, or separately or separately and sequentially, wherein the sequential administration is close in time or remote in time, for the manufacture of a medicamentation for the prevention of an early recurrency of migraine.

10. A pharmaceutical kit comprising at least two separate unit dosage forms (A) and (B):
(A) one of which comprises a composition containing an inhibitor of CGRP and optionally a pharmaceutically acceptable carrier;
(B) one of which comprises a composition containing one or more cyclooxygenase-2 inhibitors, and optionally a pharmaceutically acceptable carrier.
